Europäisches Patentamt

(19) European Patent Office

Office européen des brevets

(11) Veröffentlichungsnummer: **0 152 038**
**B1**

(12) # EUROPÄISCHE PATENTSCHRIFT

(45) Veröffentlichungstag der Patentschrift:
12.07.89

(51) Int. Cl.⁴: **A 61 K 9/32**

(21) Anmeldenummer: **85101097.5**

(22) Anmeldetag: **02.02.85**

(54) Arzneimittelüberzug.

(30) Priorität: **15.02.84 DE 3405378**

(43) Veröffentlichungstag der Anmeldung:
**21.08.85 Patentblatt 85/34**

(45) Bekanntmachung des Hinweises auf die Patenterteilung:
**12.07.89 Patentblatt 89/28**

(84) Benannte Vertragsstaaten:
**AT BE CH DE FR GB IT LI NL SE**

(56) Entgegenhaltungen:
**EP-A-0 064 485**

**PHARM. IND., Band 34, Nr. 11a, 1972, Seiten 894-899, Editio Cantor Verlag, Aulendorf, DE; K. LEHMANN et al.: "Anwendung wässriger Kunststoffdispersionen zum Überziehen von Arzneiformen"
CHEMICAL ABSTRACTS, Band 85, Nr. 12, 20. September 1976, Seite 342, Zusammenfassung Nr. 83190r, Columbus, Ohio, US; J.P. DELPORTE et al.: "Effect of the formulation of enterosoluble preparations on the drug bioavailability. Part. 4. Effect of the pH of dissolution of the applied enteric polymer on the in vivo absorption characteristics of acetylsalicylic acid tablets. Conclusions.", & J. PHARM. BELG. 1976, 31(3), 263-76**

(73) Patentinhaber: **Röhm GmbH, Kirschenallee Postfach 4242, D-6100 Darmstadt 1 (DE)**

(72) Erfinder: **Lehmann, Klaus, Dr., Schillerstrasse, D-6101 Rossdorf 1 (DE)**
Erfinder: **Dreher, Dieter, Hermannstädter Weg 34, D-6100 Darmstadt (DE)**
Erfinder: **Götz, Harry, Gernsheimerstrasse 97, D-6146 Alsbach- Hähnlein (DE)**

## Beschreibung

Die Erfindung bezieht sich auf Überzugsmittel für Arzneimittel in Form einer wäßrigen Dispersion und auf die damit überzogenen Arzneimittel.

### Stand der Technik

Überzugsmittel für Arzneitabletten, enthaltend einen wasserunlöslichen, filmbildenden Kunststoff in Form einer wäßrigen Dispersion und eine wasser- oder alkalilösliche Substanz, sind aus der DE-C-1 617 351 bekannt. Für magensaftresistente Überzüge wird eine alkalilösliche Substanz mitverwendet, die im alkalischen Milieu aus dem Tablettenüberzug herausgelöst wird und Poren hinterläßt, durch die danach der Wirkstoff ausdiffundieren kann. Als alkalilösliche Substanz werden z. B. Fettsäuren vorgeschlagen. Die Wirkstofffreisetzung beginnt, wenn die Tablette in ein wäßriges Milieu von einem pH-Wert eintritt, bei dem sich die alkalilösliche Substanz auflöst.

Aus der gleichen Druckschrift ist es auch bekannt, in den Überzug makromolekulare, wasserlösliche Substanzen einzubringen, wie Polyäthylenglykole, die unabhängig vom pH-Wert des Milieus löslich sind. Der Einsatz von alkalilöslichen makromolekularen Substanzen war noch nicht bekannt, jedoch war zu erwarten, daß sie ebenso wie niedermolekulare alkalilösliche Substanzen die Diffusionsdurchlässigkeit des Überzugs bei einem pH-Wert herbeiführen, bei dem sie löslich sind.

Die Kombination eines dispergierten Acrylesterpolymerisats mit nicht wasserlöslichen Celluloseäthern ist aus der EP-A-52 075 bekannt. Sie ergibt Arzneimittelüberzüge, die eine verzögerte Wirkstofffreisetzung bewirken.

Gemäß DE-A-3 127 237 wird eine wäßrige Dispersion eines magensaftresistenten Überzugsmittels mit Polyäthylenglykol und Polyvinylpyrrolidon kombiniert und ergibt Arzneimittelüberzüge, die im Darmsaft schnell zerfallen.

Thiele und Pflegel haben in "Pharmazie" 1981, S. 858 - 859 und 1983, S. 43 - 45 über Tablettenüberzüge aus wäßrigen Dispersionen, enthaltend 98 bis 99 Gew.-% eines schwach carboxylgruppenhaltigen hydrophoben Acryl-/Methacrylesterpolymerisats und 1 bis 2 Gew.-%, jeweils auf den Gesamtpolymerisatgehalt bezogen, eines hydrophilen Mischpolymerisats aus Acryl-/Methacrylsäure und Acryl-/Methacrylsäurealkylestern, berichtet. Der Zusatz des letztgenannten Polymermaterials bewirkt zwar eine Steigerung der Diffusionsdurchlässigkeit, aber keine schnelle Wirkstofffreigabe im alkalischen Bereich. Bei Zusätzen von mehr als 2 Gew.-% des hydrophilen Polymerisats erwies sich der Überzug als instabil. Eine Abhängigkeit der Durchlässigkeit vom pH-Wert wurde nicht festgestellt. Vielmehr erfolgt die Wirkstofffreigabe allein durch Diffusion durch die porenfreie Membran, weitgehend unabhängig vom pH-Wert.

Aus der DE-C-2 135 073 sind Arzneimittelüberzugsdispersionen von Polymeren, die 10 bis 55 Gew.-% carboxylgruppenhaltige Monomerbausteine enthalten, bekannt. Sie ergeben Überzüge auf Arzneiformen, die sich bereits im schwach alkalischen Milieu der oberen Darmabschnitte bei hohem Carboxylgruppengehalt schnell, bei niedrigem Carboxylgruppengehalt langsamer auflösen.

### Aufgabe und Lösung

Die Palette von wäßrig dispergierten Arzneimittelüberzügen war durch solche Mittel zu ergänzen, die bei mäßig erhöhter Temperatur filmbildend sind und die Überzüge ergeben, die magensaftresistent sind und erst im stärker alkalischen Darmsaft, dort aber schnell, den Wirkstoff freisetzen.

In organisch gelöster Form stehen Überzugsmittel bereit, die bei jedem gewünschten pH-Wert im physiologischen Bereich eine schnelle oder langsame Wirkstoffabgabe bewirken. Die Freigabecharakteristik läßt sich durch den Gehalt an Carboxylgruppen des Polymerisats in weiten Bereichen steuern. Dieses Prinzip ließ sich nicht auf wäßrige Überzugsmitteldispersionen übertragen. Man erreicht eine schnelle Wirkstofffreisetzung bei pH 5,5 mit einer Dispersion eines Mischpolymerisats aus gleichen Teilen Äthylacrylat und Methacrylsäure. Setzt man jedoch den Säuregehalt auf 30 Gew.-% herab, so wird der Wirkstoff beim gleichen pH-Wert, aber wesentlich langsamer freigesetzt. Eine Verschiebung der raschen Freisetzung in einen höheren pH-Bereich ließ sich auch nicht durch eine härtere Polymerisateinstellung, etwa durch einen teilweisen Ersatz der Acrylesterkomponente durch Methacrylester, erreichen. Durch diese Maßnahme geht lediglich die Fähigkeit zur Filmbildung verloren.

Es wurde nun überraschend gefunden, daß sich der Freigabebereich magensaftresistenter Überzüge zu höheren pH-Werten verschieben läßt, wenn man zum Überziehen von Arzneiformen eine wäßrige Überzugsmitteldispersion einsetzt, die als Überzugs- oder Bindemittel dispergierte Latexteilchen

A) eines zwischen pH 5 und 8 wasserlöslichen carboxylgruppenhaltigen Polymeren und
B) eines nicht wasserlöslichen filmbildenden Polymeren,

verwendet, wobei das Gewichtsverhältnis der Gesamtmengen der Latexteilchen A und B zwischen 60 : 40 und 5 : 95 liegt.

### Vorteile der neuen Überzüge

Die Freigabecharakteristik der erfindungsgemäß hergestellten Überzüge im Vergleich zu anderen Überzügen geht aus der anhängenden Graphik anschaulich hervor. Sie zeigt die Freisetzung des Wirkstoffes Chininsulfat aus Tabletten,

die mit verschieden Überzügen I bis VII überzogen und einem Milieu mit allmählich steigendem pH-Wert ausgesetzt worden sind. Damit
wird die Passage durch den Darmtrakt simuliert.

In der nachfolgenden Übersicht werden für die
Bestandteile der Mischpolymerisate folgende
Abkürzungen benutzt (% bedeutet Gewichtsprozent)

EA = Äthylacrylat
MMA = Methylmethacrylat
MA
= Methacrylsäure

Folgende Emulsionspolymerisate wurden untersucht und, soweit nicht anders angegeben, als
Dispersion bzw. Dispersionsgemisch zur Filmbildung eingesetzt.

I) 50 % EA, 50 % MA
II) 70 % EA, 30 % MA
III) 70 % EA, 30 % MA, Filmbildung aus
organischer Lösung
IV) 3 Teile (50 % EA, 50 % MA) + 7 Teile (50
% EA, 50 % MMA)
V) 3 Teile (50 % MMA, 50 % MA) + 7 Teile
(50 % EA, 50 % MMA)
VI) 3 Teile (60 % MMA, 40 % MA) + 7 Teile
(50 % EA, 50 % MMA)
VII) 3 Teile (70 % MMA, 30 % MA) + 7 Teile
(50 % EA, 50 % MMA).

Erwünscht war ein möglichst steiler Anstieg
der Freigabekurve von einem möglichst nahe an
der Null-Linie liegenden zu einem möglichst
hohen Wert. Die Forderung nach Magensaftresistenz kann als erfüllt angesehen werden,
wenn die Wirkstofffreigabe bei pH-Werten unter
4 innerhalb einer Stunde unter 5 % der eingeschlossenen Wirkstoffmenge bleibt. Wie die
Graphik zeigt, läßt sich dieses Verhalten bei allen
im Darmtrakt auftretenden pH-Werten über 5,5
durch die erfindungsgemäßen Überzugsmittel
erreichen.

Der Freigabe-pH-Wert läßt sich durch Variation
der carboxylgruppenhaltigen Komponente des
Überzugsmittels gezielt einstellen, während die
filmbildende Komponente in allen Versuchen
nach Art und Menge unverändert blieb. Es hat
sich gezeigt, daß die Filmbildung durch die von
IV nach VII zunehmend härter werdende A-
Komponente nicht beeinträchtigt wird. Daher
kann die B-Komponente ganz nach den Erfodernissen des Filmbildungsvorganges gewählt werden.

Es ist auffällig, daß das Freigabeverhalten der
erfindungsgemäßen Überzüge erheblich von den
Befunden abweicht, die Thiele und Pflegel an
Membranen aus 98 - 99 % hydrophoben und 1 bis
2 % hydrophilen, carboxylgruppenhaltigen Polymerisaten, die aus Dispersionen erzeugt worden waren, erhalten haben. Während diese
Membranen porenfrei blieben, weisen elektronenmikroskopische Untersuchungen an den
erfindungsgemäßen Überzügen darauf hin, daß die carboxylgruppenhaltige Polymerkomponente
A ab einem bestimmten pH-Wert unter Bildung
von Poren aus dem Überzug herausgelöst wird.
Dadurch steigt die Freigabegeschwindigkeit
sprunghaft an. Dieses Verhalten war um so
weniger zu erwarten, als Thiele und Pflegel keine
pH-Abhängigkeit der Freigabecharakteristik fanden.

Anwendung

Die neuen Überzugsmittel eignen sich für die
Herstellung von Arzneiformen, die den Magen
unverändert passieren und ihren Wirkstoff
schnell in einem eng begrenzten, durch seinen
pH-Wert gekennzeichneten Darmabschnitt freisetzen sollen, unter Verwendung einer wäßrigen
Überzugsmitteldispersion.

Das carboxylgruppenhaltige Polymere

liegt in Form von in einer Wasserphase dispergierten Latexteilchen vor. Die Herstellung derartiger Latizes ist in der DE-C-2 135 073 und der DE-
A-3 134 222 beschrieben. Es ist jedoch nicht
erforderlich, daß die Dispersion dieses Polymeren selbst filmbildend ist. Daher können hartmachende Comonomere, wie niedere Methacrylsäureester, einen höheren Anteil des
Polymerisats bilden, als wenn die Dispersion
allein als Überzugsmittel verwendet würde.

Das carboxylgruppenhaltige Polymere muß
wenigstens in einem Teil des pH-Bereichs zwischen 5 und 8 wasserlöslich sein, kann aber im
unteren Teil dieses Bereichs noch wasserunlöslich sein. Damit es in Wasser dispergierbar ist,
muß es wenigstens unter pH 5 wasserunlöslich
sein. Oberhalb pH 8 ist es in der Regel wasserlöslich, jedoch hat diese Eigenschaft keine Bedeutung für die Erfindung. Die wäßrige Dispersion
der Latexteilchen A und B hat auf jeden Fall einen
pH-Wert, bei dem das carboxylgruppenhaltige
Polymere ungelöst ist.

In der Regel wird das Polymere durch radikalische Emulsionspolymerisation von Vinylmonomeren in wäßriger Phase hergestellt. Ein Teil der
Vinylmonomeren, vorzugsweise 10 bis 70, insbesondere 25 bis 55 Gew.-%, enthält wenigstens
eine Carboxylgruppe. Bevorzugte Vinylmonomere
dieser Art sind Acryl- und Methacrylsäure, jedoch
sind auch Malein-, Fumar-, Croton- oder Itakonsäure einsetzbar. Der verbleibende Teil der
Vinylmonomeren ist frei von Carboxylgruppen
und kann aus Estern der genannten Carbonsäuren, insbesondere den Alkylestern mit 1 bis 8 C-
Atomen im Alkylrest, Acryl- oder Methacrylnitril,
Styrol, $\alpha$-Methylstyrol, Vinyltoluol, Vinylchlorid
oder Vinylestern von Fettsäuren, wie Vinylacetat,
bestehen. Hydrophilierende neutrale Comonomere, wie Acrylamid, Methacrylamid, Vinylpyrrolidon oder Hydroxyalkylester der Acryl- oder
Methacrylsäure können in begrenzten Mengen
am Aufbau der Emulsionspolymerisate beteiligt
sein. Sie führen zu einer gewissen Diffusionsdurchlässigkeit des Überzugs schon bei
niedrigen pH-Werten, was nur in Sonderfällen erwünscht ist.

Der Anteil der carboxylgruppenhaltigen Monomeren wird jeweils so bemessen, daß das Polymerisat im Bereich zwischen pH 5 und 8 wasserlöslich ist und der Wirkstoff beim gewünschten pH-Wert freigesetzt wird. Wird das Polymere A allein untersucht, so erweist sich seine Lösungsgeschwindigkeit vom Carboxylgruppengehalt abhängig. Hydrophile Comonomere wirken sich steigernd, hydrophobe Comonomere verzögernd auf die Lösungsgeschwindigkeit aus. Als wasserlöslich werden im Sinne der Erfindung solche Polymeren angesehen, die sich in Form vcn 10 - 20 µm dicken Filmen in künstlichem Darmsaft von pH 7,5 unter mäßigem Rühren innerhalb von höchstens 60 min auflösen. Verzögerte Wasserlöslichkeit wirkt sich in den erfindungsgemäßen Überzügen durch eine Verschiebung des Freigabepunktes zu höheren pH-Werten aus.

Die Lösungsgeschwindigkeit hängt auch vom Molekulargewicht ab. Der Gewichtsmittelwert des Molekulargewichts liegt im allgemeinen nicht über 500.000 und vorzugsweise im Bereich von 50.000 bis 300.000.

### Das filmbildende Polymere

liegt gleichfalls in Form von wäßrig dispergierten Latexteilchen vor und wird ebenfalls bevorzugt durch radikalische Emulsionspolymerisation geeigneter Vinylmonomerer erzeugt. Filmbildende wäßrige Polymerdispersionen sind in großer Zahl im Handel und ihre Herstellung ist aus zahlreichen Druckschriften allgemein bekannt.

Als filmbildend werden die Emulsionspolymerisate bezeichnet, wenn sie unter den üblichen Anwendungsbedingungen von wäßrigen Arzneimittelüberzugsdispersionen einen zusammenhängenden, an dem überzogenen Kern fest haftenden Film ergeben. In der Regel tritt eine solche Filmbildung schon bei Raumtemperatur ein, jedoch kann auch eine höhere Trocknungstemperatur angewandt werden. Das filmbildende Polymere wird in der Regel so ausgewähhlt, daß es in Form des wäßrigen Latex eine Mindestfilmbildungstemperatur nach DIN 53787 nicht über 60°C, vorzugsweise nicht über 40°C hat. Diese Bedingung ist im allgemeinen erfüllt, wenn die dynamische Einfriertemperatur ($T_{\lambda max}$-Wert nach DIN 53445) nicht über 80°C, vorzugsweise nicht über 60°C liegt.

Die Emulsionspolymerisate können aus den gleichen Vinylmonomeren aufgebaut sein wie die carboxylgruppenhaltigen Polymeren, jedoch liegt der Anteil an carboxylgruppenhaltigen Comonomeren wesentlich niedriger oder fehlt völlig. Auch hydrophile Comonomere sind nur in so begrenzter Menge einpolymerisiert, daß das Polymere im physiologischen pH-Bereich des Magen-Darm-Traktes von 1 bis 8 nicht wasserlöslich ist. Wasserlöslichkeit erst oberhalb pH 8 würde die Brauchbarkeit des Polymeren nicht ausschließen.

Die Filmbildungsfähigkeit setzt einen ausreichenden Anteil von "weichen" Comonomeren am Aufbau des Polymerisats voraus.

Hierzu gehören in erster Linie die Alkylester der Acrylsäure. Sie bilden in der Regel einen Anteil im Bereich von 40 bis 80 Gew.-% des Polymerisats, jedoch soll ihr Anteil nicht so hoch sein, daß die dynamische Einfriertemperatur unter 0°C liegt, weil andernfalls daraus hergestellte Überzüge zu weich oder sogar klebrig würden. Handelsübliche Arzneimittelüberzugsdispersionen für die Herstellung unlöslicher Überzüge auf Diffusionstabletten erfüllen diese Forderungen und sind für die Zwecke der Erfindung besonders geeignet.

### Das Überzugsmittel

wird zweckmäßig durch Mischen der Latizes vom Typ A und B hergestellt, wobei das Mischungsverhältnis der Gewichtsanteile der beiden Polymertypen zwischen 60 : 40 und 5 : 95, vorzugsweise zwischen 50 : 50 und 30 : 70 liegt. Es versteht sich von selbst, daß die zu mischenden Dispersionen miteinander verträglich sein müssen. Beispielsweise dürfen sie keine entgegengesetzt geladenen Emulgiermittel enthalten, wenn es dadurch zur Koagulation käme.

Das Mischungsverhältnis beeinflußt die Mindestfilmbildungstemperatur des Überzugsmittels. Sie liegt zwischen den Mindestfilmbildungstemperaturen der Ausgangslatizes der Polymeren A und B. Wenn sie unerwünscht hoch liegt, kann sie durch einen Zusatz von Filmbildungshilfsmitteln, die entweder als Weichmacher im Film verbleiben oder als flüchtiges Lösungsmittel bei der Trocknung entweichen, erniedrigt werden. Beispiele solche Filmbildungshilfsmittel sind Äthylenoder Propylenglykol, Glycerin, Citronensäureester und Polyäthylenglykole.

Mit zunehmendem Anteil des Polymeren A im Verhältnis zum Polymeren B steigt oberhalb des pH-Wertes, bei dem überhaupt eine Wirkstoffdurchlässigkeit auftritt, die Freisetzungsgeschwindigkeit für den in der überzogenen Arzneiform eingeschlossenen Wirkstoff. Während bei niedrigen Gehalten des Polymeren A die Überzugsmembran zwar durchlässig wird, aber erhalten bleibt, zerfällt sie bei höheren Gehalten an Polymeren A bald nach Erreichen des pH-Wertes, bei dem sie durchlässig wird, vor allem wenn der überzogene Kern durch Quellung eine Sprengwirkung entfaltet.

Die Polymeren A und B bilden in der Regel einen Anteil von 10 bis 40 Gew.-% des wäßrigen Überzugsmittels. Der Rest entfällt auf Wasser, darin gelöste Emulgiermittel und eventuelle Zusätze.

Neben den Polymeren A und B kann das Überzugsmittel übliche gelöste oder suspendierte Hilfsmittel und Zusätze enthalten. Außer den schon erwähnten Filmbildungshilfsmitteln kommen als Zusätze z. B. Konservierungsmittel, Verdickungsmittel, Glanzmittel, Farbstoffe und Pigmente in Betracht.

Die Viskosität des flüssigen Überzugsmittels liegt zweckmäßigerweise im Bereich von 10 bis 100 cP. Sein pH-Wert liegt unter 6, in der Regel zwischen 2 und 5.

### Die überzogene Arzneiform

Erfindungsgemäß können alle Arzneiformen überzogen werden, die magensaftresistent sein müssen und den eingeschlossenen Wirkstoff im Darmtrakt bei einem vorbestimmten pH-Wert verhältnismäßig schnell freisetzen sollen. Im Regelfall werden wenigstens 80 % des Wirkstoffes innerhalb 60 min freigesetzt.

Es können Tabletten, Drageekerne, Pillen, Granulate, Kristalle, Pulver gegebenfalls sogar Gelatinekapseln überzogen werden. Man kann auch Granulate oder Pellets unter Verwendung der erfindungsgemäßen Mittel nach einem üblichen Granulierverfahren herstellen. Die Granulate können ihrerseits überzogen oder zu Tabletten verpreßt werden.

Die Überzugsverfahren entsprechen denjenigen, die bei herkömmlichen Arzneimittelüberzugsmitteldispersionen angewendet werden können. Bevorzugt sind Kesseldragierverfahren, bei denen das Überzugsmittel portionsweise oder kontinuierlich auf die rotierenden Arzneiformen aufgegossen oder aufgesprüht wird. Dabei wird in der Regel Warmluft zum Trocknen aufgeblasen. Vorteilhaft ist weiterhin das Wirbelschichtverfahren, das vorzugsweise bei einer Lufttemperatur von 40 bis 60°C durchgeführt wird.

Die pH-abhängige Freigabecharakteristik tritt bei Schichtdicken von 10 bis 30 μm besonders deutlich in Erscheinung. Bei der Beschichtung von Granulaten, Partikeln und Kristallen entspricht dies einem Lackauftrag von 10 bis 20 Gew.-%, bei Tabletten, Dragees oder Kapseln einem Lackauftrag von 3 bis 5 Gew.-%, jeweils bezogen auf das Gewicht des überzogenen Kerns. Unterhalb dieses Bereiches ist mit einer zunehmend zeitabhängigen statt pH-abhängigen Freigabe, oberhalb dieses Bereichs mit zunehmend verzögerter Freigabe beim pH-Wert des Auflösungsbereichs zu rechnen.

**Beispiel 1**

141 g einer 30-%-igen Dispersion eines Copolymerisats aus gleichen Teilen Methacrylsäure und Äthylacrylat (42 g Trockensubstanz) wurden mit 328 g einer ebenfalls 30-%-igen Dispersion eines Copolymerisats aus Äthylacrylat und Methylmethacrylat im Verhältnis 2 : 1 (98 g Trockensubstanz) vermischt und anschließend noch eine Suspension von 70 g Talkum in 514 g Wasser zugefügt. Die Mischung hat pH 5,7.

3 kg Chininsulfat-Tabletten (Einzelgewicht 206,5 mg, Durchmesser 8 mm, Höhe 3,7 mm) wurden in einem Dragierkessel von 35 cm Durchmesser bei 40 UpM durch Einblasen von Warmluft von 65 - 70°C auf etwa 32°C vorgewärmt und anschließend kontinuierlich unter weiterem Einblasen von Trockenluft mit der wäßrigen Polymeremulsion besprüht. Es wurde hierzu eine Luftdrucksprühpistole mit einem Düsendurchmesser von 1,0 mm und einem Sprühdruck von 0,8 bar verwendet. Die Polymeremulsion wurde der Sprühpistole über eine Schlauchpumpe zugeführt und damit die Sprühgeschwindigkeit auf ca. 9 g pro min eingestellt. Die Gesamtsprühzeit betrug 2 Std. Anschließend wurden die überzogenen Tabletten noch 2 Std. bei 40°C im Umlufttrockenschrank nachgetrocknet.

Bei der Prüfung im USP-Zerfallstester waren die Tabletten zunächst 60 min magensaftresistent, auch nach weiteren 30 min in einer Pufferlösung von 5,5 trat weder Zerfall noch Wirkstoffabgabe ein. In Pufferlösung pH 6,0 erfolgte dann schnelle Wirkstoffabgabe (siehe Kurve IV) und alle Tabletten zerfielen innerhalb von 12 - 16 min.

**Beispiel 2**

141 g einer 30-%-igen Dispersion eines Copolymerisats aus gleichen Teilen Methacrylsäure und Methylmethacrylat (42 g Trockensubstanz) wurden mit 328 g einer ebenfalls 30-%-igen Dispersion eines Copolymerisats aus Äthylacrylat und Methylmethacrylat im Verhältnis 2 : 1 (98 g Trockensubstanz) vermischt und anschließend noch eine Suspension von 70 g Talkum in 514 g Wasser zugefügt. Der pH-Wert betrug 6,0.

3 kg Chininsulfat-Tabletten wurden wie in Beispiel 1 überzogen.

Bei der Prüfung der überzogenen Tabletten im USP-Zerfallstester waren diese Tabletten ebenfalls 60 min magensaftresistent, auch nach weiteren 30 min in einer Prüflösung von pH 5,5 und auch weiteren 30 min in einer Prüflösung pH 6,0 traten noch kein Zerfall und keine Wirkstoffabgabe ein. Erst als anschließend eine Pufferlösung von pH 6,5 eingebracht wurde, zerfielen die Tabletten innerhalb von 2 - 11 min unter schneller Wirkstoffabgabe (siehe Kurve V).

**Beispiel 3**

141 g einer 30-%-igen Dispersion eines Copolymerisats aus Methacrylsäure und Methylmethacrylat im Verhältnis 1 : 2 (42 g Trockensubstanz) wurden mit 328 g einer ebenfalls 30-%-igen Dispersion eines Copolymerisats aus Äthylacrylat und Methylmethacrylat im Verhältnis 2 : 1 (98 g Trockensubstanz) vermischt und anschließend noch eine Suspension von 70 g Talkum in 514 g Wasser zugefügt. Der ph-Wert lag bei 6,6.

3 kg Chininsulfat-Tabletten wurden wie in Beispiel 1 überzogen. Die überzogenen Tabletten waren im USP-Zerfallstester 60 min magensaftresistent und zeigten auch bei den pH-Wert 5,5, 6,0, 6,5 und 7,0 weder Zerfall noch nennenswerte Wirkstoffabgabe, wenn sie jeweils 30 min in diesen Prüflösungen bewegt wurden. Erst bei pH 7,5 zerfielen die Tabletten innerhalb von 11 - 16 min unter schneller Wirkstoffabgabe (Kurve VII).

**Beispiel 4**

Es wurden 1000 g eines 30-%-igen Emulsionspolymerisats aus Äthylacrylat und Methylmethacrylat im Verhältnis 2 : 1 vorgelegt (300 g Trockensubstanz) und mit einer Lösung von 10 g Polyoxyäthylensorbitanmonooleat (Tween 80®) in 20 g Wasser vermischt. Dazu wurden 1000 g eines 30-%-igen Emulsionspolymerisates aus gleichen Teilen Methacrylsäure und Äthylacrylat gegeben (300 g Trockensubstanz) und anschließend noch eine Aufschlämmung von 151 g Talkum in 582 g Wasser zugesetzt, wobei noch 1 g einer Silikonantischaumemulsion beigemischt werden. Die Mischung hatte pH 5,6.

1 kkg Theophyllin-Granulat einer Korngröße von 0,3 - 0,8 mm wurde in einem Wirbelschichtgerät (Uniglatt®) durch Einblasen von Warmluft aufgewirbelt, auf etwa 40°C vorgewärmt und unter Aufrechterhaltung eines Warmluftstromes von 40°C mit Hilfe einer in das Wirbelbett hineinragenden Düse besprüht. Düsenöffnung 1,2 mm, Sprühdruck 1,8 bar. Die Sprühgeschwindigkeit, die über eine Schlauchpumpe reguliert wurde, betrug 11 g/min, die Gesamtsprühzeit 131 min. Der Gesamtlackauftrag entspricht einer Gewichtszunahme des Granulats von 20 %. Es wurden Proben auch nach 10 und 15 % Lackauftrag entnommen. Schon nach 10 % Lackauftrag ist eine deutliche Verzögerung der Wirkstoffauflösung im Magensaft zu beobachten.

Das mit 15 % Lackgemisch überzogene Granulat ist im USP-Paddle-Gerät 120 min magensaftresistent, d.h. die Wirkstoffabgabe liegt unter 5 %; anschließend tritt schon bei pH 5,5 schnelle Wirkstoffabgabe ein.

Das mit 20 % Lackgemisch überzogene Granulat ist ebenfalls magensaftresistent, zeigt dann bei pH 5,5 zunächst nur eine langsame Wirkstoffabgabe, die bei pH 6,5 stark ansteigt.

**Beispiel 5**

1000 g eines 30-%-igen Emulsionspolymerisates aus Äthylacrylat und Methylmethacrylat im Verhältnis 2 : 1 (300 g Trockensubstanz) wurden nach Zugabe von 10 g Polyoxyäthylensorbitanmonooleat in 20 g Wasser mit 100 g eines 30-%-igen Emulsionspolymerisats aus gleichen Teilen Methacrylsäure und Äthylacrylat (30 g Trockensubstanz) vermischt und aus 685 g dieser Mischung eine Sprührezeptur mit einer Aufschlämmung von 150 g Talkum in 600 g Wasser angesetzt. Beim Zusammenrühren der Bestandteile wurde zur Unterdrückung von Schaumbildung noch 1 g Silikonantischaumemulsion zugesetzt. Die Suspension hatte pH 6,8.

Diese Sprührezeptur wurde wie in Beispiel 4 beschrieben auf 1 kg Theophyllin-Granulat aufgesprüht. Die Freigabekurven nach 5 - 15 % Lackauftrag zeigen ab 7,5 % Lackauftrag eine annähernd lineare Wirkstoffabgabe.

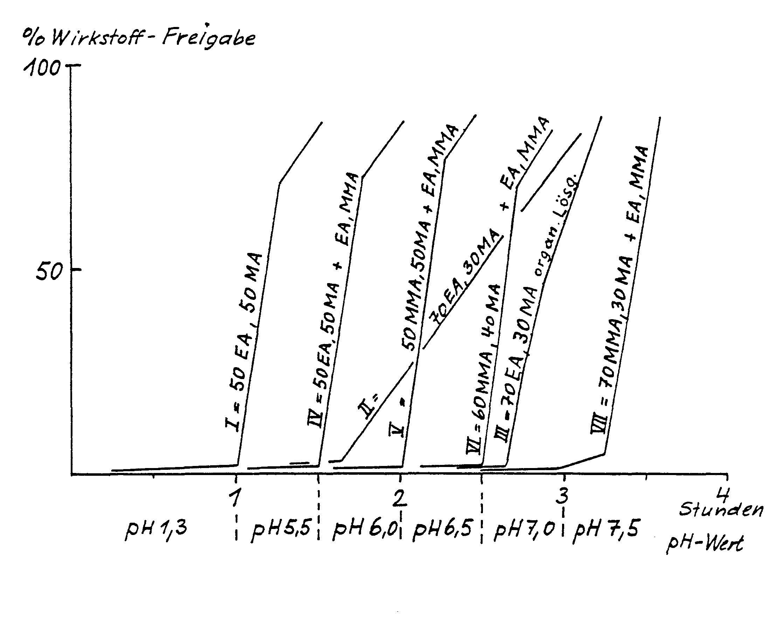

**Patentansprüche**

1. Wäßrige Dispersion eines Überzugsmittels für Arzneiformen, enthaltend dispergierte Latexteilchen

A) eines zwischen pH 5 und 8 wasserlöslichen, carboxylgruppenhaltigen Polymeren, und

B) eines nicht wasserlöslichen, filmbildenden Polymeren,

<u>dadurch gekennzeichnet,</u>
daß das Gewichtsverhältnis der Gesamtmenge der Latexteilchen A und B zwischen 60 : 40 und 5 : 95 liegt.

2. Wäßrige Dispersion nach Anspruch 1, dadurch gekennzeichnet, daß die Polymeren A und B Emulsionspolymerisate von Vinylmonomeren sind.

3. Wäßrige Dispersion nach Anspruch 2, dadurch gekennzeichnet, daß das carboxylgruppenhaltige Polymere 10 bis 70 Gew.-% Einheiten der Acryl- und/oder Methacrylsäure enthält.

4. Wäßrige Dispersion nach den Ansprüchen 2 und 3, dadurch gekennzeichnet, daß die Polymeren A und B einpolymerisierte Einheiten von Alkylestern der Acryl- und/oder Methacrylsäure enthalten.

5. Wäßrige Dispersion nach den Ansprüchen 1 bis 4, gekennzeichnet durch eine dynamische Einfriertemperatur des filmbildenden Polymeren zwischen 10 und 60°C.

6. Wäßrige Dispersion nach den Ansprüchen 1 bis 5, gekennzeichnet durch einen Gehalt an den Polymeren A und B von 10 bis 40 Gew.-%

7. Verwendung einer wäßrigen Dispersion nach den Ansprüchen 1 bis 6, zum Überziehen von Arzneiformen.

8. Verfahren zur Herstellung magensaftresistent überzogener Arzneiformen durch Überziehen mit einer wäßrigen Arzneimittelüberzugsdispersion und Trocknen, dadurch gekennzeichnet, daß eine wäßrige Dispersion gemäß den Ansprüchen 1 bis 6 aufgebracht wird.

9. Verfahren nach Anspruch 8, dadurch gekennzeichnet, daß die Trocknung der überzogenen Arzneiformen mit Luft einer Temperatur unter 60°C durchgeführt wird.

10. Magensaftresistente Arzneiform, enthaltend einen Überzug, der als Überzugs- oder Bindemittel die Polymeren A und B gemäß Anspruch 1 im Gewichtsverhältnis zwischen 60 : 40 und 5 : 95 enthält.

11. Arzneiform nach Anspruch 10, gekennzeichnet durch einen Überzug einer Dicke von 10 bis 30 µm.

12. Arzneiform nach Anspruch 11, gekennzeichnet durch die Form von überzogenen Granulaten, Partikeln oder Kristallen mit einem Lackauftrag von 10 bis 20 Gew.-%, bezogen auf das Gewicht des überzogenen Kerns.

13. Arzneiform nach Anspruch 11, gekennzeichnet durch die Form von überzogenen Tabletten, Dragees oder Kapseln mit einem Lackauftrag von 3 bis 5 Gew.-%, bezogen auf das Gewicht des überzogenen Kerns.

## Claims

1. Aqueous dispersion of a coating composition for pharmaceutical preparations, containing dispersed latex particles

A) of a carboxyl group-containing polymer which is water-soluble at between pH 5 and 8, and
B) a water-insoluble film-forming polymer

characterised in that the weight ratio of the total quantity of latex particles A and B is between 60 : 40 and 5 : 95.

2. Aqueous dispersion according to claim 1, characterised in that the polymers A and B are emulsion polymers of vinyl monomers.

3. Aqueous dispersion according to claim 2, characterised in that the carboxyl group-containing polymer contains from 10 to 70 % by weight of units of acrylic and/or methacrylic acid.

4. Aqueous dispersion according to claims 2 and 3, characterised in that the polymers A and B contain units of alkyl esters of acrylic and/or methacrylic acid polymerised therein.

5. Aqueous dispersion according to claims 1 to 4, characterised by a dynamic freezing temperature of the film-forming polymer of between 10 and 60°C.

6. Aqueous dispersion according to claims 1 to 5, characterised by a content of polymers A and B from 10 to 40 % by weight.

7. Use of an aqueous dispersion according to claims 1 to 6 for coating pharmaceutical preparations.

8. Process for preparing pharmaceutical compositions provided with a coating resistant to gastric juices, by coating with an aqueous pharmaceutical coating dispersion and drying, characterised in that an aqueous dispersion as claimed in claims 1 to 6 is applied.

9. Process according to claim 8, characterised in that the coated pharmaceutical preparations are dried with air at a temperature of below 60°C.

10. Pharmaceutical preparation resistant to gastric juices, containing a coating which contains, as the coating or binding agent, the polymers A and B as claimed in claim 1 in a weight ratio of between 60 : 40 and 5 : 95.

11. Pharmaceutical preparation according to claim 10, characterised by a coating with a thickness of from 10 to 30 µm.

12. Pharmaceutical preparation according to claim 11, characterised in that it takes the form of coated granules, particles or crystals with an application of lacquer from 10 to 20 % by weight, based on the weight of the coated core.

13. Pharmaceutical preparation according to claim 11, characterised in that it takes the form of tablets, coated tablets or capsules provided with a coating of lacquer of from 3 to 5 % by weight, based on the weight of the coated core.

## Revendications

1. Dispersion aqueuse d'un agent d'enrobage pour formes médicamenteuses, contenant des particules dispersées de latex

A) d'un polymère contenant des groupements carboxyle, soluble dans l'eau à un pH compris entre 5 et 8 et
B) d'un polymère filmogène non soluble dans l'eau,
caractérisée en ce que le rapport pondéral de la quantité totale des particules de latex A et B se situe entre 60 : 40 et 5 : 95.

2. Dispersion aqueuse selon la revendication 1, caractérisée en ce que les polymères A et B sont des produits de polymérisation en émulsion de monomères vinyliques.

3. Dispersion aqueuse selon la revendication 2, caractérisée en ce que le polymère contenant des groupements carboxyle contient 10 à 70 % en poids d'unités acide acrylique et/ou méthacrylique.

4. Dispersion aqueuse selon la revendication 2 ou 3, caractérisée en ce que les polymères A et B contiennent en liaison polymère des unités ester alkylique de l'acide acrylique et/ou méthacrylique.

5. Dispersion aqueuse selon l'une quelconque des revendications 1 à 4, caractérisée par une température de durcissement dynamique du polymère filmogène comprise entre 10 et 60°C.

6. Dispersion aqueuse selon l'une quelconque des revendications 1 à 5, caractérisée par une teneur en polymères A et B de 10 à 40 % en poids.

7. Utilisation d'une dispersion aqueuse selon l'une quelconque des revendications 1 à 6 pour l'enrobage de formes médicamenteuses.

8. Procédé de préparation de formes médicamenteuses enrobées gastro-résistantes par enduction avec une dispersion aqueuse d'enrobage médicamenteux et séchage, caractérisé en ce qu'on applique une dispersion aqueuse selon l'une quelconque des revendications 1 à 6.

9. Procédé selon la revendication 8, caractérisé en ce que le séchage des formes médicamenteuses enrobées est effectué avec de l'air à une température inférieure à 60°C.

10. Forme médicamenteuse gastro-résistante, comportant un revêtement qui contient, en tant qu'agent d'enrobage ou liant, les polymères A et B selon la revendication 1 dans un rapport pondéral compris entre 60 : 40 et 5 : 95.

11. Forme médicamenteuse selon la revendication 10, caractérisée par un enrobage d'une épaisseur de 10 à 30 μm.

12. Forme médicamenteuse selon la revendication 11, caractérisée par la forme de granulés, de particules ou de cristaux enrobés, sur lesquels est appliquée une couche de vernis de 10 à 20 % en poids par rapport au poids du noyau enrobé.

13. Forme médicamenteuse selon la revendication 11, caractérisée par la forme de comprimés, de dragées ou de capsules enrobés, sur lesquels est appliquée une couche de vernis de 3 à 5 % en poids par rapport au poids du noyau enrobé.